(19) Europäisches Patentamt
European Patent Office
Office européen des brevets

(11) Publication number: 0 331 195 A2

# (12) EUROPEAN PATENT APPLICATION

(21) Application number: 89103780.6

(22) Date of filing: 03.03.89

(51) Int. Cl.4: C07C 97/00 , C07D 333/22 , C07D 295/10 , C07D 307/46 , C07D 207/333

(30) Priority: 04.03.88 JP 51977/88

(43) Date of publication of application: 06.09.89 Bulletin 89/36

(84) Designated Contracting States: DE IT NL SE

(71) Applicant: NIPPON SHINYAKU COMPANY, LIMITED
14, Kisshoin Nishinosho Monguchicho
Minami-ku Kyoto-shi Kyoto 601(JP)

(72) Inventor: Kise, Masahiro
Tominokoji Higashiiru Anayakoji
Nakakyo-ku Kyoto 604(JP)
Inventor: Yoshimoto, Yoshihiko
2-13-14 Higashiyakura
Kusatsu-shi Shige-ken 525(JP)
Inventor: Fujisawa, Hiroshi
17-6 Seifucho
Otsu-shi 520-02(JP)
Inventor: Sasaki, Yasuo
1-9-2 Miharu Shinomachi
Kameoka-shi 621(JP)
Inventor: Yasufuku, Shoji
2-1-2-104 Oeda Nishishimbayashicho
Nishikyo-ku Kyoto 610-11(JP)

(74) Representative: Wey, Hans-Heinrich, Dipl.-Ing.
Patentanwälte Wey & Partner
Widenmayerstrasse 49
D-8000 München 22(DE)

## (54) Acylphenol derivatives.

(57) Acylphenol derivatives represented by the general formula (I)

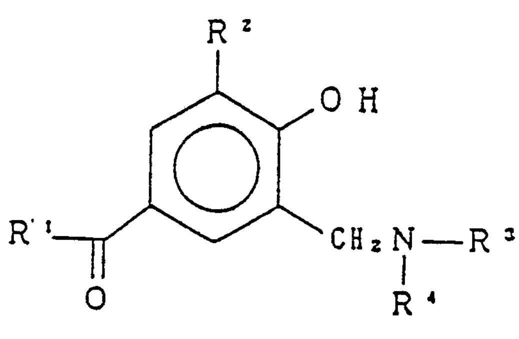

( I )

and physiologically-acceptable salts thereof are claimed, wherein $R^1$ is cycloalkyl, substituted or unsubstituted aryl or

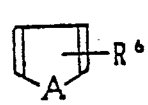

EP 0 331 195 A2

in which A is oxygen, sulfur or $NR^5$ ($R^5$ is hydrogen or alkyl) and $R^6$ is hydrogen, alkyl or halogen; $R^2$ is alkyl or cycloalkyl; and $R^3$ and $R^4$, which are same or different, are hydrogen, substituted or unsubstituted alkyl, alkenyl, alkynyl or cycloalkyl or $R^3$ and $R^4$ are bonded forming cyclic amino together with the adjacent nitrogen atom.

# Acylphenol Derivatives

3. Detailed Description of the Invention:

(Technical Field)

The present invention relates to acylphenol derivatives represented by the following general formula (I)

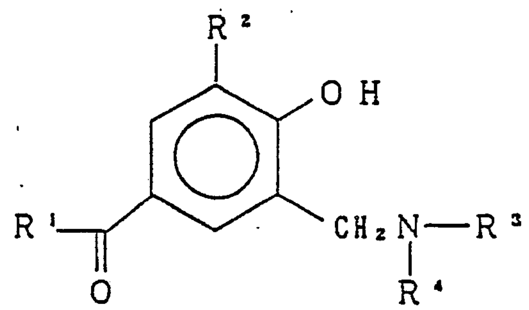

( I )

in which $R^1$ is cycloalkyl, substituted or unsubstituted aryl or

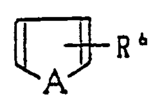

wherein A is oxygen,sulfur or $NR^5$ ($R^5$ is hydrogen or alkyl ) and $R^6$ is hydrogen, alkyl or halogen; $R^2$ is alkyl or cycloalkyl; and $R^3$ and $R^4$(which are same or different)are hydrogen, substituted or unsubstituted alkyl, alkenyl, alkynyl or cycloalkyl or they are bonded and form cyclic amino together with the adjacent nitrogen atom.

As fully illustrated hereinafter, the compounds of the present invention exhibit analgesic, antiinflammatory and antipyretic action and are useful as pharmaceuticals. (Prior Art)

Various types of steroidal and nonsteroidal pharmaceuticals have been used as antiinflammatory/analgesic agents. Though steroidal ones exhibits excellent antiinflammatory action and show remarkable effect to arthritis, rheumatism, etc., their continued used causes serious side effects such as hormonic action and is a big disturoance on the therapy.

On the contrary, nonsteroidal ones exhibits excellent antiinflammatory action too,but their continued use causes gastrointestinal disturbances and they have undesired situation in terms of different viewpoint from steroidal ones.

In the meanwhile it has been known already that some derivatives of 4-acyl-2,6-di-tert-butylphenol exhibit an inhibitory action to cyclooxygenase and inhibit carrageenin-induced edema. For instance, 3,5-di-tert-butyl-4-hydroxy-phenyl 2-thienyl ketone which is similar to the compounds of present invention (known as R-830; cf. Agents and Actions, vol.12, page 5, 1982) has been reported to exhibit inhibitory action against carrageenin-induced edema.

(Problems to be solved by the Invention)

The present inventors have conducted studies to find out substances which satisfy both of the following two requirements. (1) The above-given disadvantages can be fully overcome; and (2) they exhibit sufficient antiinflammatory action. Accordingly, objects of the present invention are the above-given two points.

(Means to solve the Problems)

The characteristic feature of the present invention exists in the structure per se of the compound represented by the formula (I). The compounds of the present invention are novel being not disclosed in the prior art literatures yet and, as mentioned later, they exhibit excellent pharmaceutical activity with low toxicity.

Compounds of the present invention will now be further illustrated as hereunder.

The cycloalkyl represented by $R^1$ in the general formula (I) is preferably that with 5 to 7 carbon atoms such as, for example, cyclopentyl, cyclohexyl, cycloheptyl, etc. The aryl preferred is that with 6 to 10 carbon atoms such as, for example, phenyl, alpha-naphthyl, beta-naphthyl, etc. The aryl may have one or more substituent(s) at the position(s) which is/are not restricted. Examples of such substituents are alkyl, halogen, trifluoromethyl, etc. As to the alkyl here, straight or branched one with 1 to 4 carbon atom(s) is preferred and is, for example, methyl, ethyl, n-propyl, isopropyl, n-butyl, isobutyl, sec-butyl, tert-butyl, etc. Examples of the halogen are chlorine, fluorine, bromine, iodine, etc. Examples of the

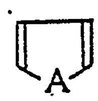

are 2-thienyl, 3-thienyl, 2-furyl, 3-furyl, 2-pyrrolyl, 3-pyrrolyl, etc. which may be substituted. There is no particular restriction as to the numbers and positions of such substituents. Examples of such substituents are alkyl, halogen, etc. Examples of such alkyl and halogen are the same as those which are exemplified hereinabove already.

Examples of the alkyl represented by $R^2$ may be the same as those exemplified already. Examples of cycloalkyl may be the same as those exemplified already too.

The alkyl represented by $R^3$ and $R^4$ is preferably that with 1 to 8 carbon atom(s) and is straight or branched such as, for example, methyl, ethyl, n-propyl, isopropyl, n-butyl, isobutyl, sec-butyl, tert-butyl, n-pentyl, isopentyl, n-hexyl, isohexyl, n-heptyl, isoheptyl, n-octyl, isooctyl, etc. Examples of substituents for such an alkyl are hydroxy, acyloxy with 2 to 5 carbon atoms (such as acetyloxy, propionyloxy, butyryloxy, etc.), carboxyl and carbamoyl. Preferred alkenyl is straight or branched one having 2 to 6 carbon atoms such as, for example, vinyl, allyl, isopropenyl, 2-methallyl, 2-butenyl, 3-butenyl, etc.

Preferred alkynyl is straight or branched one having 2 to 6 carbon atoms such as, for example, ethynyl, 1-propynyl, 2-propynyl, etc. Examples of the cycloalkyl are those which are exemplified in the description for $R^1$ hereinabove.

Preferred cyclic amino formed by $R^3$ and $R^4$ together with adjacent nitrogen atom is that having 5 to 7 members. Heteroatoms such as oxygen, sulfur, nitrogen, etc. may contain therein as member(s) of the ring. Examples of such cyclic amino groups are pyrrolidino, piperidino, piperazino, morpholino, thiomorpholino, homopiperazino, etc.

Those cyclic amino group may be substituted and there is no particular limitation as to the numbers and positions of the substituent(s). Examples of such substituents are $C_{1-4}$ alkyl, hydroxyalkyl, hydroxy, etc.

Examples of the salts of the compounds of the present invention are those with mineral acids such as hydrochloric acid, sulfuric acid, nitric acid, phosphoric acid, etc. and with organic acids such as oxalic acid, tartaric acid, maleic acid, benzenesulfonic acid, etc.

The compounds of the present invention may, for example, be manufactured by the following step:

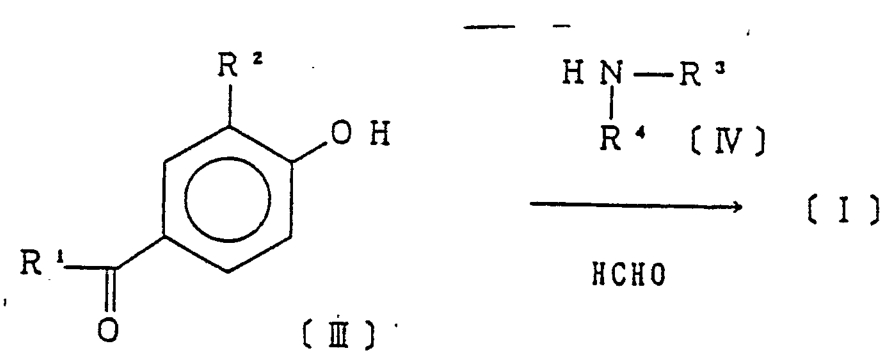


in which the meanings of $R^1$, $R^2$, $R^3$ and $R^4$ are the same as those defined hereinbefore already.

Thus, p-acylphenol derivative (III) is made to react with secondary amine (IV) to afford the compound (I) of the present invention. This reaction is a so-called Mannich reaction and is conducted in the presence of an aldehyde in the absence of solvent or presence of a solvent which is inert to the reaction. As to the solvent for the reaction, that which is commonly used in the reaction of this type such as, for example, alcohols (e.g. methanol, ethanol, propanol, butanol, etc.), ethers (e.g. tetrahydrofuran, dioxane, etc.), aprotic solvent (e.g. acetonitrile, N,N- diemthylformamide, dimethyl sulfoxide, sulfolane, etc., water or a mixture thereof may be used. As to the aldehydes, those exhibiting the role as formaldehyde during Mannich reaction may be used. They are, for example, paraformaldehyde, formalin, methylal, ethylal, piperidinomethylphthalimide, hexamethylenetetramine, etc.

The amount of the aldehyde and the secondary amine may be sufficient if they are 1 to 10 times more in terms of moles to (III). The secondary amine may be used in a form of a salt. The reaction temperature may be usually at 0 to 100 °C. The reaction time may vary depending upon the type of the material and the aldehyde used, reaction temperature, etc. and usually it may be sufficient for 1 to 40 hours.

The starting materials (III) may include novel compounds and may, for example, be manufactured by the following means as disclosed in the referential examples which will be given later.

(1)

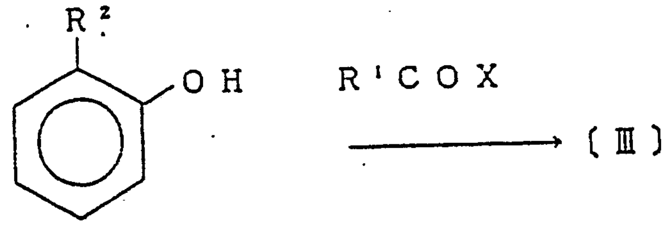

(II)

in which the meanings of $R^1$ and $R^2$ are the same as those defined before and X stands for halogen.

Thus, phenol derivative (II) is made to react with acid halide to give (III). This reaction is Friedel-Crafts reaction or something like that and can be carried out in a solvent inert to the reaction in the presence of Lewis acid if required.

As to the solvent for the reaction, that which is commonly used in the reaction of this type is used. For example, carbon disulfide, nitrobenzene, halogenated hydrocarbons (e.g. chlorobenzene, dichloromethane, dichloroethane, trichloroethane, tetrachloroethane, etc.), aromatic hydrocarbons (e.g. benzene, toluene, xylene, etc.), and the like may be used.

Examples of the Lewis acids applicable are tin chloride, titanium chloride, zinc chloride, iron chloride, boron tribromide, boron trifluoride, concentrated sulfuric acid, etc. Among those, the use of titanium tetrachloride and stannic chloride is particularly preferred.

The amount of the Lewis acid and the acid chloride is 1 to 6 times more in terms of moles, preferably from equimolar to a little excess, to the substance (II). The reaction temperature is usually preferred from -20 to + 140 °C The reaction time may vary depending upon the materials, catalyst, reaction temperature, etc. but, usually, it may be sufficient for 4 to 12 hours.

(2)

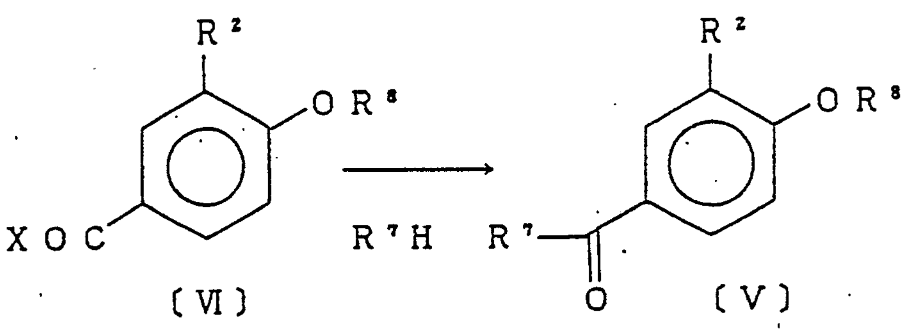

(VI) (V)

in which the meanings of $R^2$ and X are the same as those defined before; $R^8$ is a protective group for hydroxyl; $R^7$ is aryl or an aromatic heterocycle represented by

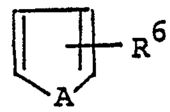

and A and $R^6$ are the same as those defined already.

An acid halide (VI) and a compound of a formula $R^7H$ are made to react to afford (V). Removal of a protective group from (V) easily gives (III). This reaction can be carried out under the condition which is substantially same as that in the above reaction (1).

Examples of the protective group represented by $R^8$ are those which are known as protective groups for hydroxyl such as, for example, alkyl, aralkyl, acyl, etc.

In removing the protective group, conventional means depending upon the particular protective group may be applied. For example, when $R^8$ is alkyl, dealkylation using acid may be applied. When $R^8$ aralkyl, reductinve dearalkylation or hydrolysis using acid may be applied. When $R^8$ is acyl, hydrolysis using an alkali may be applied.

The resulting compound (I) manufactured as such may be isolated/purified, either in a form of free base or in a form of acid-addition salt, by concentration, a change of the pH of the liquid, dissolution in another solvent, extraction with a solvent, crystallization, recrystallization, fractional distillation, chromatography, and the like.

When the compound of the present invention is administered as pharmaceuticals, the present invention compound per se or that in a form of a pharmaceutical composition containing the compound in a concentration of, for example, 0.1-99.5% (preferably, 0.5-90%) in a pharmaceutically-acceptable and nontoxic/inert carrier is given to animals including human being.

As to a carrier, one or more auxiliary agent(s) for pharmaceutical prescriptions such as diluent, filler, etc. in a form of solid, semi-solid or liquid. It is preferred that pharmaceutical composition is administered in a form of unit dose. The pharmaceutical composition of the present invention may be administered per os, into tissues, to local places (e.g. via skin) or via rectum. Needless to say, the pharmaceutical preparation type suitable for each administration means is applied. Oral administration is, for example, preferred.

The dose as analgesic, antipyretic or antiinflammatory agent is recommended to ajust taking age, body weight, and other parameters of the patient, administering route, type and degree of the diseases, etc. into consideration but, usually, it is preferred to administer 0.1-1,000 mg/day, more preferably 30-600 mg/day, for human being. In some cases, less dose will be sufficient while, in some other cases, more dose will be necessary. It is also possible that the dose may be divided and given several times a day.

(Examples)

The present invention will be further illustrated by way of the following referential examples as well as examples of the present invention.

Referential Example 1. 3-(1,1-Dimethylethyl)-4-hydroxybenzophenone.

Titanium chloride (20.86 g) was dissolved in 80 ml of 1,2-dichloroethane, 14.7 g of benzoyl chloride was dropped thereinto with ice-cooling and stirring, then 15.0 g of 2-tert-butylphenol was dropped thereinto at the same temperature, and the mixture was stirred for 5 hours at ambient temperature. The reaction solution was poured over into ice water, extracted with ether, the extract was washed with water, aqueous solution (saturated) of sodium bicarbonate and water successively, dried with magnesium sulfate, ether was evaporated therefrom, and to the residue was added n-hexane whereupon desired compound was crystallised. This was collected by filtration and dried to give 11.2 g of crystals.

Referential Example 2. 3-(1,1-Dimethylethyl)-4-hydroxy-phenyl 2-thienyl ketone.

Titanium tetrachloride (865.5 g) was dissolved in 2.3 liters of 1,2-dichloroethane and 502.2 g of 2-

thenoyl chloride was dropped thereinto during 20 minutes with ice cooling. Then 517.4 g of 2-tert-butylphenol was dropped during 30 minutes and the mixture was stirred for 1.5 hours at room temperature. The reaction solution was poured over 2 kg of ice and extracted with 6.0 liters of ether. The extract was washed with water, then with aqueous solution of sodium bicarbonate and with saturated aqueous solution of sodium chloride, dried, and concentrated in vacuo. To the concentrate was added n-hexane, the mixture was allowed to stand overnight, the crystals separated out therefrom were filtered, and washed with n-hexane to give 576.2 g of the desired compound as pale yellow crystals. M.p. 180-181 ° C (benzene).

Similarly prepared were the following compounds.

3-(1,1-Dimethylethyl)-4-hydroxy-4′-methylbenzophenone, m.p. 209-210 ° C (ethyl acetate);
4′-Chloro-3-(1,1-dimethylethyl)-4-hydroxybenzophenone, m.p. 205-206 ° C (ethyl acetate);
3′-Chloro-3-(1,1-dimethylethyl)-4-hydroxybenzophenone, m.p. 173-174 ° C (ligroin/ethyl acetate);
3-(1-Methylpropyl)-4-hydroxybenzophenone, m.p. 95-96 ° C (ligroin/ethyl acetate);
3-(1,1-Dimethylethyl)-4′-fluoro-4-hydroxybenzophenone, m.p. 203-204 ° C (ethyl acetate);
1-Naphthyl 3-(1,1-dimethylethyl)-4-hydroxyphenyl ketone, m. p. 177-178 ° C (ethyl acetate);
Cyclohexyl 3-(1,1-dimethylethyl)-4-hydroxyphenyl ketone, m.p. 142-143 ° C (ethyl acetate/n-hexane);
3-Cyclohexyl-4-hydroxybenzophenone, m.p. 199-200 ° C (ethyl acetate);
3-(1,1-Dimethylethyl)-2′-fluoro-4-hydroxybenzophenone, m.p. 152-153 ° C (n-hexane/ethyl acetate);
2-Naphthyl 3-(1,1-dimethylethyl)-4-hydroxyphenyl ketone, m.p. 189-190 ° C (ethyl acetate);
3-(1,1-Dimethylethyl)-4-hydroxyphenyl 2-furyl ketone, m.p. 146-148 ° C;
3-Cyclohexyl-4-hydroxyphenyl 2-thienyl keton, m.p. 203-204 ° C (ether/n-hexane);
3-(1-Methylethyl)-4-hydroxyphenyl 2-thienyl ketone, m.p. 132-133 ° C (n-hexane);
3-(1-Methylpropyl)-4-hydroxyphenyl 2-thienyl ketone, m.p. 90-92 ° C (n-hexane);
3-(1,1-Dimethylethyl)-4-hydroxy-2′-trifluoromethylbenzophenone, m.p. 160-161 ° C (n-hexane); and
3-(1,1-Diemthylethyl)-4-hydroxy-2′,6′-dichlorobenzophenone, m.p. 229-230 ° C (n-hexane).

Referential Example 3. 3-(1,1-Dimethylethyl)-4-hydroxyphenyl 5-bromo-2-thienyl ketone.

3-(1,1-Dimethylethyl)-4-acetoxybenzoyl chloride (5.4 g) and 4.5 g of 2-bromothiophene were dissolved in 60 ml of 1,2-dichloroethane and, with ice cooling and stirring, 5.8 g of stannic chloride was dropped thereinto. The reaction solution was stirred overnight at room temperature, poured over ice water and extracted with ether. The extract was washed with water and then with saturated aqueous solution of sodium chloride, ether was evaporated therefrom, and the residue was heated to reflux for 3 hours with 100 ml of 10% aqueous solution of sodium hydroxide and 50 ml of ethanol. After the reaction, concentrated hydrochloric acid was added to the reaction solution and the acidified solution was extracted with ether. The extract was washed with saturated aqueous solution of sodium chloride and dried with magnesium sulfate. Ether was evaporated therefrom and the residue was subjected to a silica gel column chromatography followed by eluting with chloroform. The fraction containing the desired product was concentrated and the resulting oil was crystallized from chloroform/n-hexane to give 4 g of crystals, m.p. 197-199 ° C

Similarly prepared were the following compounds.

3-(1,1-Dimethylethyl)-4-hydroxyphenyl 5-methyl-2-thienyl ketone, m.p. 198-200 ° C (chloroform/n-hexane);
3-(1,1-Dimethylethyl)-4-hydroxyphenyl 4-methyl-2-thienyl ketone, m.p. 165-166 ° C (n-hexane); and
3-(1,1-Dimethylethyl)-4-hydroxyphenyl 3-methyl-2-thienyl ketone, m.p. 153-155 ° C (ethanol/water).

Referential Example 4. 3-(1,1-Dimethylethyl)-4-hydroxyphenyl 5-chloro-2-thienyl ketone.

3-(1,1-Dimethylethyl)-4-methoxybenzoyl chloride (6.3 g) was dissolved in 55 ml of 1,2-dichloroethane and, with ice cooling and stirring, 6.0 g of titanium tetrachloride and then 3.7 g of 2-chlorothiophene were dropped thereinto. The mixture was stirred overnight at room temperature, poured over ice water and extracted with ether. The extract was washed with water, aqueous solution of sodium hydroxide and saturated aqueous solution of sodium chloride successively and dried with magnesium sulfate. Ether was evaporated therefrom and the resulting residue was heated with stirring for 2 hours at 220-230 ° C with 24 g of pyridine hydrochloride. After the reaction, diluted hydrochloric acid was added thereto, extracted with ether, the extract was washed with water, dried with magnesium sulfate, evaporated and the resulting oil was crystallised from chloroform and n-hexane and further recrystallized from ethanol/water to give 5.1 g of desired product, m.p. 200-201 ° C

Similarly prepared was 3-(1,1-dimethylethyl)-4-hydroxyphenyl 3-chloro-2-thienyl ketone, m.p. 158-159 °C.

Referential Example 5. 3-(1,1-Dimethylethyl)-4-hydroxyphenyl 1-methyl-2-pyrrolyl ketone.

3-(1,1-Dimethylethyl)-4-acetoxybenzoyl chloride (5.1 g) and 4.9 g of 1-methylpyrrole were dissolved in 150 ml of xylene and the mixture was heated at 140 °C for one day with stirring. 1-Methylpyrrole (4.0 g) was added to the reaction solution and the mixture was heated for two days with stirring at the same temperature as above. Xylene was evaporated therefrom, the residue was subjected to a silica gel column chromatography, and eluted with chloroform to give the desired acetate as an oil.

To this were added 30 ml of ethanol and 15 ml of 10% aqueous solution of soidum hydroxide, the mixture was heated with refluxing for 1 hour, acidified with hydrochloric acid, extracted with ether, the extract was washed with saturated aqueous solution of sodium bicarbonate, dried with magnesium sulfate, ether was evaporated therefrom, and the resulting oil was crystallized from chloroform and n-hexane to afford 3.2 g of the desired product, m.p. 182-183 °C.

Example 1. 3-(N,N-Dimethylaminomethyl)-5-(1,1-dimethylethyl)-4-hydroxybenzophenone.

3-(1,1-Dimethylethyl)-4-hydroxybenzophenone (2.5 g), 1.71 g of dimethylamine hydrochloride, 0.78 g of sodium hydroxide and 0.68 g of paraformaldehyde were dissolved in 30 ml of ethanol and the mixture was heated to reflux for 16 hours. Ethanol was evaporated therefrom. Water was added to the residue and the crystals separated out therefrom were filtered and recrystallized from 90% ethanol to give 2.1 g of the desired product, m.p. 129 °C.

Elem. Anal. ($C_{20}H_{25}NO_2$)

| | | | |
|---|---|---|---|
| Calcd (%) | C : 77.14 | H : 8.09 | N : 4.50 |
| Found (%) | C : 77.27 | H : 8.44 | N : 4.53 |

Example 2. 3-(N,N-Diethylaminomethyl)-5-(1,1-dimethylethyl)-4-hydroxybenzophenone hydrochloride.

3-(1,1-Dimethylethyl)-4-hydroxybenzophenone (1.27 g), 0.65 g of diethylamine and 35% aqueous solution of formaldehyde were dissolved in 30 ml of ethanol and the solution was heated to reflux with stirring for 8 hours. Ethanol was evaporated therefrom, hydrochloric acid was added to the residue, the mixture was washed with ether, the hydrochloric acid layer was made alkaline with aqueous solution of sodium bicarbonate, and the oil separated out therefrom was extracted with ether. The extract was washed with water, dried with magnesium sulfate, and ether was evaporated therefrom to give 0.86 g of oil. This was dissolved in 20 ml of ether, the solution was allowed to stand for 1 hour with ethereal hydrochloric acid, crystals separated out therefrom were filtered and recrystallized from ethanol to give 0.5 g of crystals of m.p. 143-145 °C.

Elem. Anal. ($C_{22}H_{29}NO_2 \cdot HCl$)

| | | | |
|---|---|---|---|
| Calcd (%) | C : 70.29 | H : 8.04 | N : 3.72 |
| Found (%) | C : 70.28 | H : 8.05 | N : 3.77 |

Example 3. 3-(N,N-Dimethylaminoethyl)-5-(1,1-dimethylethyl)-4-hydroxy-4′-methylbenzophenone.

3-(1,1-Dimethylethyl)-4-hydroxy-4′-methylbenzophenone (2.68 g) was suspended in 45 ml of ethanol

and heated with stirring to reflux for 8 hours with 1.80 g of 50% aqueous solution of dimethylamine and 1.7 g of 35% aqueous solution of formaldehyde. Insoluble matters were filtered off, the filtrate was evaporated to dryness in vacuo and the residue was crystallized from ethanol to give 2.36 g of white crystals, m.p. 123-124.5° C.

Elem. Anal.($C_{21}H_{27}NO_2$)

| | | | |
|---|---|---|---|
| Calcd (%) | C : 77.50 | H : 8.36 | N : 4.30 |
| Found (%) | C : 77.40 | H : 8.68 | N : 4.18 |

Example 4. 3- (1,1-Dimethylethyl)-4-hydroxy-5-[N-(2-hydroxyethyl)-N-methylaminomethyl]phenyl 2-thienyl ketone.

3-(1,1-Dimethylethyl)-4-hydroxyphenyl 2-thienyl ketone (22.0 g) was dissolved in 440 ml of ethanol and heated to reflux for 6 hours with 12.7 g of N-methylethanolamine, 14.5 g of 35% aqueous solution of formaldehyde and 50.8 g of acetic acid. After the reaction, the solvent was evaporated therefrom. To the residue was added saturated aqueous solution of sodium bicarbonate, the mixture was extracted with chloroform thrice, the extract was dried with magnesium sulfate, the solvent was evaporated therefrom, the resulting yellow oil was subjected to a column chromatography (500 g of silica gel being used; eluates were (1) ethylacetate/n-hexane in 3:1 and (2) the same in 1:1) and isolated/purified product was recrystallized from methanol/water to give 23.33 g of pale yellow crystals, m.p. 98-100° C (methanol/water).

Elem. Anal.($C_{19}H_{25}NO_3S$)

| | | | |
|---|---|---|---|
| Calcd (%) | C : 65.68 | H : 7.25 | N : 4.03 |
| Found (%) | C : 65.64 | H : 7.26 | N : 4.00 |

Similarly prepared were the compounds of the following examples.

Example 5. 3-(1,1-Dimethylethyl)-4-hydroxy-5-morpholinomethylbenzophenone hydrochloride.

M.p. 237-239° C (ethanol)

Elem. Anal.($C_{22}H_{27}NO_3 \cdot HCl$)

| | | | |
|---|---|---|---|
| Calcd (%) | C : 67.66 | H : 7.24 | N : 3.59 |
| Found (%) | C : 67.70 | H : 7.37 | N : 3.58 |

Example 6. 3-(1,1-Dimethylethyl)-4-hydroxy-5-(N-methylpiperazinomethyl)benzophenone dihydrochloride.

M.p. 228-230° C (ethanol)

Elem. Anal.($C_{23}H_{30}N_2O_2 \cdot 2HCl \cdot \frac{1}{2}H_2O$)

Calcd (%) C : 61.94 H : 7.86 N : 6.24

Found (%) C : 61.60 H : 7.42 N : 6.25

Example 7. 3-(N,N-Diethylaminomethyl)-5-(1,1-dimethylethyl)-4-hydroxy-4′-methylbenzophenone.

M.p. 97-99° C(ethanol).

Elem. Anal.($C_{23}H_{31}NO_2$)

Calcd (%) C : 78.15 H : 8.84 N : 3.96

Found (%) C : 78.21 H : 8.53 N : 4.14

Example 8. 3-(1,1-Diemthylethyl)-4-hydroxy-5-morpholinomethyl-4′-methylbenzophenone.

M.p. 131-133° C (ethanol)

Elem. Anal.($C_{23}H_{29}NO_3$)

Calcd (%) C : 75.17 H : 7.95 N : 3.81

Found (%) C : 75.00 H : 8.19 N : 3.94

Example 9. 4′-Chloro-3-(N,N-dimethylaminomethyl)-5-(1,1-dimethylethyl)-4-hydroxybenzophenone hydrochloride.

M.p. 226-228° C (decomposition) (ethanol)

Elem. Anal.($C_{20}H_{24}ClNO_2 \cdot HCl$)

Calcd (%) C : 62.83 H : 6.59 N : 3.66

Found (%) C : 62.77 H : 6.81 N : 3.76

Example 10. 4′-Chloro-3-(N,N-diethylaminomethyl)-5-(1,1-dimethylethyl)-4-hydroxybenzophenone.

M.p. 95-96.5° C (ethanol)

Elem. Anal. ($C_{22}H_{28}ClNO_2$)

Calcd (%) C : 70.67 H : 7.55 N : 3.75

Found (%) C : 70.63 H : 7.63 N : 3.87

Example 11. 4′-Chloro-3-(1,1-dimethylethyl)-4-hydroxy-5-morpholinomethylbenzophenone.

M.p. 165-166° C (ethanol)

Elem. Anal. ($C_{22}H_{28}ClNO_3$)

Calcd (%) C : 68.12 H : 6.76 N : 3.61

Found (%) C : 68.17 H : 6.94 N : 3.71

Example 12. 4′-Chloro-3-(1,1-dimethylethyl-4-hydroxy-5-(N-methylpiperazinomethyl)benzophenone.

M.p. 131-132° C (ethanol)

Elem. Anal. ($C_{23}H_{29}ClN_2O_2$)

Calcd (%) C : 68.90 H : 7.29 N : 6.99

Found (%) C : 68.92 H : 7.43 N : 7.08

Example 13. 3′-Chloro-3-(N,N-dimethylaminomethyl)-5-(1,1-dimethylethyl)-4-hydroxybenzophenone.

M.p. 143-145° C (ethanol)

Elem. Anal. ($C_{20}H_{24}ClNO_2$)

Calcd (%) C : 69.45 H : 6.99 N : 4.05

Found (%) C : 69.44 H : 7.22 N : 4.14

Example 14. 3-(N,N-Dimethylaminomethyl)-5-(1,1-dimethylethyl)-4-hydroxyphenyl 2-thienyl ketone.

M.p. 132-134° C (ethanol)

Elem. Anal. ($C_{18}H_{23}NO_2S$)

Calcd (%) C : 68.11 H : 7.30 N : 4.41

Found (%) C : 68.04 H : 7.34 N : 4.41

Example 15. 3-(N,N-Dimethylaminomethyl)-5-(1,1-dimethylethyl)-5-hydroxyphenyl 2-thienyl ketone hydrochloride.

M.p. 211-212° C

Elem. Anal. ($C_{18}H_{23}NO_2S \cdot HCl$)

| | | | |
|---|---|---|---|
| Calcd (%) | C : 61.09 | H : 6.84 | N : 3.96 |
| Found (%) | C : 61.07 | H : 6.89 | N : 4.25 |

Example 16. 3-(1,1-Dimethylethyl)-4-hydroxy-5-pyrrolidinomethylphenyl 2-thienyl ketone.

M.p. 96-97° C (ethanol/water)

Elem. Anal. ($C_{20}H_{25}NO_2S$)

| | | | |
|---|---|---|---|
| Calcd (%) | C : 69.94 | H : 7.34 | N : 4.08 |
| Found (%) | C : 69.98 | H : 7.52 | N : 4.07 |

Example 17. 3-(1,1-Dimethylethyl)-4-hydroxy-5-piperidinomethylphenyl 2-thienyl ketone.

M.p. 132-133° C (ethanol)

Elem. Anal. ($C_{21}H_{27}NO_2S$)

| | | | |
|---|---|---|---|
| Calcd (%) | C : 70.55 | H : 7.61 | N : 3.92 |
| Found (%) | C : 70.48 | H : 7.69 | N : 3.88 |

Example 18. 3-(1,1-Dimethylethyl)-4-hydroxy-5-morpholinomethylphenyl 2-thienyl ketone.

M.p. 147.5-148.5° C (ethanol-water)

Elem. Anal. ($C_{20}H_{25}NO_3S$)

| | | | |
|---|---|---|---|
| Calcd (%) | C : 66.82 | H : 7.01 | N : 3.90 |
| Found (%) | C : 66.83 | H : 6.94 | N : 3.93 |

Example 19. 3-(1,1-Dimethylethyl)-4-hydroxy-5-(4-methylpiperazinomethyl)phenyl 2-thienyl ketone.

M.p. 118-119° C (ethanol/water)

Elem. Anal. ($C_{21}H_{28}N_2O_2S$)

| | | | |
|---|---|---|---|
| Calcd (%) | C : 67.71 | H : 7.58 | N : 7.52 |
| Found (%) | C : 67.78 | H : 7.56 | N : 7.54 |

Example 20. 3-(N,N-Diethylaminomethyl)-5-(1,1-dimethylethyl)-4-hydroxyphenyl 2-thienyl ketone hydrochloride.

M.p. 144-145° C

Elem. Anal. ($C_{20}H_{27}NO_2S \cdot HCl$)

| | | | |
|---|---|---|---|
| Calcd (%) | C : 62.89 | H : 7.39 | N : 3.67 |
| Found (%) | C : 62.76 | H : 7.29 | N : 3.64 |

Example 21. 3-(1,1-Dimethylethyl)-4-hydroxy-5-thiomorpholinomethylphenyl 2-thienyl ketone.

M.p. 165-166° C (ethanol)

Elem. Anal. ($C_{20}H_{25}NO_2S_2$)

| | | | |
|---|---|---|---|
| Calcd (%) | C : 63.93 | H : 6.71 | N : 3.73 |
| Found (%) | C : 64.17 | H : 6.55 | N : 3.69 |

Example 22. 3-(N,N-Dimethylaminomethyl)-5-(1,1-dimethylethyl)-4-hydroxyphenyl 5-methyl-2-thienyl ketone.

M.p. 119-120° C (ethanol/water)

Elem. Anal. ($C_{19}H_{25}NO_2S$)

| | | | |
|---|---|---|---|
| Calcd (%) | C : 68.85 | H : 7.60 | N : 4.23 |
| Found (%) | C : 68.84 | H : 7.72 | N : 4.21 |

Example 23. 3-(N,N-Dimethylaminomethyl)-5-(1,1-dimethylethyl)-4-hydroxyphenyl 5-chloro-2-thienyl ketone.

M.p. 137-138° C (ethanol)

Elem. Anal. ($C_{18}H_{22}ClNO_2S$)

Calcd (%) C : 61.44 H : 6.30 N : 3.98

Found (%) C : 61.42 H : 6.34 N : 3.22

Example 24. 3-(N,N-Dimethylaminomethyl)-5-(1,1-dimethylethyl)-4-hydroxyphenyl 5-bromo-2-thienyl ketone.

M.p. 150-152° C (ethanol)

Elem. Anal. ($C_{18}H_{22}BrNO_2S$)

Calcd (%) C : 54.55 H : 5.59 N : 3.53

Found (%) C : 54.93 H : 5.59 N : 3.55

Example 25. 3-(1,1-Dimethylethyl)-4-hydroxy-5-bis(2-hydroxyethyl)aminomethylphenyl 2-thienyl ketone.

M.p. 108-110° C (ethanol/water)

Elem. Anal. ($C_{20}H_{27}NO_4S$)

Calcd (%) C : 63.63 H : 7.21 N : 3.71

Found (%) C : 63.47 H : 7.74 N : 4.15

Example 26. 3-(N,N-Dimethylaminomethyl)-5-(1,1-dimethylethyl)-4-hydroxyphenyl 1-methyl-2-pyrrolyl ketone.

M.p. 173.5-174.5° C (ethanol)

Elem. Anal. ($C_{19}H_{26}N_2O_2$)

Calcd (%) C : 72.58 H : 8.33 N : 8.91

Found (%) C : 72.55 H : 8.39 N : 8.94

Example 27. 3-(N,N-Diethylaminomethyl)-4-hydroxy-5-(1,1-dimethylethyl)phenyl 2-furyl ketone hydrochloride.

M.p. 133-135° C (isopropanol/ether)

Elem. Anal.($C_{20}H_{27}NO_3$ · HCl)

Calcd (%) C : 65.65 H : 7.71 N : 3.83

Found (%) C : 65.30 H : 8.03 N : 4.05

Example 28. 3-(N,N-Diethylaminomethyl)-5-(1,1-dimethylethyl)-4-hydroxyphenyl 1-methyl-2-pyrrolyl ketone hydrochloride.

M.p. 170-172° C (isopropanol/ether)

Elem. Anal.($C_{21}H_{30}N_2O_2$ · HCl)

Calcd (%) C : 66.56 H : 8.25 N : 7.39

Found (%) C : 66.55 H : 8.36 N : 7.49

Example 29. 3-(N,N-Dimethylaminomethyl)-5-(1,1-dimethylethyl)-4-hydroxyphenyl 3-chloro-2-thienyl ketone.

M.p. 131-132° C (ethanol)

Elem. Anal.($C_{18}H_{22}NO_2ClS$)

Calcd (%) C : 61.44 H : 6.30 N : 3.98

Found (%) C : 61.38 H : 6.03 N : 4.08

Example 30. 3-(N,N-Dimethylaminomethyl)-5-(1,1-dimethylethyl)-4-hydroxyphenyl 3-methyl-2-thienyl ketone.

M.p. 149-150° C (ethanol)

Elem. Anal.($C_{19}H_{25}NO_2S$)

Calcd (%) C : 68.85 H : 7.60 N : 4.23

Found (%) C : 68.86 H : 7.47 N : 4.41

Example 31. 3-(N,N-Dimethylaminomethyl)-5-(1,1-dimethylethyl)-4-hydroxyphenyl cyclohexyl ketone

M.p. 149-150° C (ethanol)

Elem. Anal.($C_{20}H_{31}NO_2$)

| | | | |
|---|---|---|---|
| Calcd (%) | C : 75.66 | H : 9.84 | N : 4.41 |
| Found (%) | C : 75.81 | H : 9.84 | N : 4.63 |

Example 32. 3-(N,N-Dimethylaminomethyl)-5-(1,1-dimethylethyl)-4-hydroxyphenyl 2-naphthyl ketone.

M.p. 162.5-163.5° C (ethanol)

Elem. Anal.($C_{24}H_{27}NO_2$)

| | | | |
|---|---|---|---|
| Calcd (%) | C : 79.74 | H : 7.53 | N : 3.87 |
| Found (%) | C : 79.66 | H : 7.23 | N : 4.00 |

Example 33. 3-(N,N-Dimethylaminomethyl)-5-(1,1-dimethylethyl)-4-hydroxyphenyl 4-methyl-2-thienyl ketone hydrochloride.

M.p. 187-189° C (isopropanol)

Elem. Anal.($C_{19}H_{25}NO_2S \cdot HCl$)

| | | | |
|---|---|---|---|
| Calcd (%) | C : 62.02 | H : 7.12 | N : 3.81 |
| Found (%) | C : 61.95 | H : 7.37 | N : 3.94 |

Example 34. 3-(N,N-Diethylaminomethyl)-5-(1,1-dimethylethyl)-2′-fluoro-4-hydroxybenzophenone hydrochloride.

M.p. 162° C (ethanol)

Elem. Anal.($C_{22}H_{28}FNO_2 \cdot HCl$)

| | | | |
|---|---|---|---|
| Calcd (%) | C : 67.08 | H : 7.42 | N : 3.56 |
| Found (%) | C : 67.34 | H : 7.46 | N : 3.62 |

Example 35. 3-(N,N-Diethylaminomethyl)-5-(1,1-dimethylethyl)-4-hydroxyphenyl 2-naphthyl ketone hydrohloride.

M.p. 175-176° C (isopropanol/ether)

Elem. Anal.($C_{26}H_{31}NO_2 \cdot HCl$)

| | | | |
|---|---|---|---|
| Calcd (%) | C : 73.31 | H : 7.57 | N : 4.03 |
| Found (%) | C : 73.22 | H : 7.70 | N : 4.15 |

Example 36. 3-(N,N-Dimethylaminomethyl)-5-(1,1-dimethylethyl)-4′-fluoro-4-hydroxybenzophenone.

M.p. 112-113° C (ethanol)

Elem. Anal.($C_{20}H_{24}FNO_2$)

| | | | |
|---|---|---|---|
| Calcd (%) | C : 72.92 | H : 7.34 | N : 4.25 |
| Found (%) | C : 72.93 | H : 7.20 | N : 4.42 |

Example 37. 3-(N,N-Diethylaminomethyl)-5-(1,1-dimethylethyl)-4′-fluoro-4-hydroxybenzophenone hydrochloride.

M.p 177-178° C (isopropanol)

Elem. Anal.($C_{20}H_{28}FNO_2 \cdot HCl$)

| | | | |
|---|---|---|---|
| Calcd (%) | C : 67.08 | H : 7.42 | N : 3.56 |
| Found (%) | C : 67.14 | H : 7.46 | N : 3.75 |

Example 38. 3-(1,1-Dimethylethyl)-4-hydroxy-5-[N-(2-hydroxyethyl)-N-methylaminomethyl]-4′-fluorobenzophenone hydrochloride.

M.p. 133-134° C (isopropanol/ether)

Elem. Anal.($C_{21}H_{26}FNO_3 \cdot HCl$)

| | | | |
|---|---|---|---|
| Calcd (%) | C : 63.71 | H : 6.87 | N : 3.54 |
| Found (%) | C : 63.77 | H : 6.94 | N : 3.63 |

Example 39. 3-(N,N-Dimethylaminomethyl)-5-(1,1-dimethylethyl)-4-hydroxyphenyl 1-naphthyl ketone hydrochloride.

M.p. 210-211° C (isopropanol)

Elem. Anal. ($C_{24}H_{27}NO_2 \cdot HCl$)

Calcd (%) C : 72.44 H : 7.09 N : 3.52

Found (%) C : 72.57 H : 6.93 N : 3.55

Example 40. 3-(N,N-Diethylaminomethyl)-5-(1,1-dimethylethyl)-4-hydroxyphenyl 1-naphthyl ketone hydrochloride.

M.p. 173-174°C (isopropanol/ether)

Elem. Anal. ($C_{26}H_{31}NO_2 \cdot HCl$)

Calcd (%) C : 73.31 H : 7.57 N : 3.29

Found (%) C : 73.21 H : 7.39 N : 3.54

Example 41. 3-(1,1-Dimethylethyl)-4-hydroxy-5-morpholinomethylphenyl 1-naphthyl ketone.

M.p. 145-146°C (ethanol)

Elem. Anal. ($C_{26}H_{29}NO_3$)

Calcd (%) C : 77.39 H : 7.24 N : 3.47

Found (%) C : 77.05 H : 7.22 N : 3.66

Example 42. 3-(1,1-Dimethylethyl)-4-hydroxy-5-piperidinomethylphenyl 1-naphthyl ketone hydrochloride.

M.p. 218-219°C (ethanol)

Elem. Anal. ($C_{27}H_{31}NO_2 \cdot HCl$)

Calcd (%) C : 74.04 H : 7.38 N : 3.20

Found (%) C : 74.20 H : 7.18 N : 3.30

Example 43. 3-(1,1-Dimethylethyl)-4-hydroxy-5-[N-(2-hydroxyethyl)piperazinomethyl] benzophenone dihydrochloride.

M.p. 181-182°C (isopropanol/ether)

Elem. Anal.($C_{24}H_{32}N_2O_3 \cdot 2HCl \cdot \frac{1}{2}H_2O$)

| | | | |
|---|---|---|---|
| Calcd (%) | C : 60.24 | H : 7.37 | N : 5.85 |
| Found (%) | C : 60.28 | H : 7.49 | N : 5.88 |

Example 44. 3-(1,1-Dimethylethyl)-4-hydroxy-5-(4-hydroxypiperidinomethyl) benzophenone.

M.p. 156-157° C (ethanol)

Elem. Anal.($C_{23}H_{28}NO_3$)

| | | | |
|---|---|---|---|
| Calcd (%) | C : 75.38 | H : 7.70 | N : 3.82 |
| Found (%) | C : 75.04 | H : 7.97 | N : 4.05 |

Example 45. 3-Cyclohexyl-5-(N,N-diethylaminomethyl)-4-hydroxybenzophenone.

M.p. 106-107° C (ethanol)

Elem. Anal.($C_{24}H_{31}NO_2$)

| | | | |
|---|---|---|---|
| Calcd (%) | C : 78.86 | H : 8.55 | N : 3.85 |
| Found (%) | C : 78.54 | H : 8.72 | N : 4.28 |

Example 46. 3-(N,N-Dimethylaminomethyl)-4-hydroxy-5-(1-methylethyl)phenyl 2-thienyl ketone hydrochloride.

M.p. 170-171° C (isopropanol)

Elem. Anal.($C_{17}H_{21}NO_2S \cdot HCl$)

| | | | |
|---|---|---|---|
| Calcd (%) | C : 60.08 | H : 6.52 | N : 4.12 |
| Found (%) | C : 60.06 | H : 6.24 | N : 4.22 |

Example 47. 3-(N,N-Diethylaminomethyl)-4-hydroxy-5-(1-methylethyl)phenyl 2-thienyl ketone hydrochloride.

M.p. 168.5-169.5° C

Elem. Anal. ($C_{19}H_{25}NO_2S \cdot HCl$)

Calcd (%) C : 62.02 H : 7.12 N : 3.81

Found (%) C : 62.02 H : 7.04 N : 3.99

Example 48. 3-Cyclohexyl-5-(N,N-dimethylaminomethyl)-4-hydroxyphenyl 2-thienyl ketone.

M.p. 105-106° C (ethanol/water)

Elem. Anal. ($C_{20}H_{25}NO_2S$)

Calcd (%) C : 69.94 H : 7.34 N : 4.08

Found (%) C : 69.95 H : 7.52 N : 4.34

Example 49. 3-Cyclohexyl-5-(N,N-diethylaminomethyl)-4-hydroxyphenyl 2-thienyl ketone hydrochloride.

M.p. 204-206° C (ethanol)

Elem. Anal. ($C_{22}H_{29}NO_2S \cdot HCl$)

Calcd (%) C : 64.77 H : 7.41 N : 3.43

Found (%) C : 64.67 H : 7.51 N : 3.68

Example 50. 3-Cyclohexyl-5-(N,N-dimethylaminomethyl)-4-hydroxybenzophenone hydrochloride.

M.p. 223-225° C (ethanol)

Elem. Anal. ($C_{22}H_{27}NO_2 \cdot HCl$)

Calcd (%) C : 70.67 H : 7.55 N : 3.75

Found (%) C : 70.56 H : 7.49 N : 3.81

Example 51. 3-(N,N-Dimethylaminomethyl)-4-hydroxy-5-(1-methylpropyl)benzophenone hydrochloride.

M.p. 135-136° C (ethanol)

Elem. Anal. ($C_{20}H_{25}NO_2 \cdot HCl$)

Calcd (%) C : 69.05 H : 7.53 N : 4.03

Found (%) C : 68.98 H : 7.70 N : 4.15

Example 52. 3-(N,N-Dimethylaminomethyl)-4-hydroxy-5-(1-methylpropyl)phenyl 2-thienyl ketone hydrochloride.

M.p. 168-169 °C (isopropanol)

| Elem. Anal.($C_{18}H_{23}NO_2S \bullet HCl$) | | | |
|---|---|---|---|
| Calcd (%)<br>Found (%) | C : 61.09<br>C : 61.06 | H : 6.84<br>H : 6.69 | N : 3.96<br>N : 4.08 |

Example 53. 3-(1,1-Dimethylethyl)-4-hydroxy-5-(N-ethyl-N-methylaminomethyl)phenyl 2-thienyl ketone hydrochloride.

M.p. 202-204 °C (ether/methanol)

| Elem. Anal.($C_{19}H_{25}NO_2S \bullet HCl$) | | | |
|---|---|---|---|
| Calcd (%)<br>Found (%) | C : 62.02<br>C : 61.97 | H : 7.12<br>H : 7.02 | N : 3.81<br>N : 3.79 |

Example 54. 3-(1,1-Dimethylethyl)-4-hydroxy-5-(N-methylaminomethyl)phenyl 2-thienyl ketone.

M.p. 128-129 °C (ethanol/water)

| Elem. Anal.($C_{17}H_{21}NO_2S$) | | | |
|---|---|---|---|
| Calcd (%)<br>Found (%) | C : 67.30<br>C : 67.22 | H : 6.98<br>H : 7.00 | N : 4.62<br>N : 4.68 |

Example 55. 3-(1,1-Dimethylethyl)-4-hydroxy-5-amino-methylphenyl 2-thienyl ketone hydrochloride.

M.p. 187-188 °C (isopropanol/ether)

| Elem. Anal.($C_{16}H_{19}NO_2S \bullet HCl$) | | | |
|---|---|---|---|
| Calcd (%)<br>Found (%) | C : 58.98<br>C : 59.06 | H : 6.19<br>H : 6.09 | N : 4.30<br>N : 4.30 |

Example 56. 3-(1,1-Dimethylethyl)-4-hydroxy-5-[N-(2-hydroxyethyl)-N-methylaminomethyl]phenyl 2-thienyl ketone hydrochloride.

M.p. 176-177.5 °C (ethanol)

| Elem. Anal.($C_{19}H_{25}NO_3S \bullet HCl$) | | | |
|---|---|---|---|
| Calcd (%)<br>Found (%) | C : 59.44<br>C : 59.27 | H : 6.83<br>H : 6.78 | N : 3.65<br>N : 3.76 |

Example 57. 3-(1,1-Dimethylethyl)-4-hydroxy-5-[N-methyl-N-propargyl)aminomethyl ]phenyl 2-thienyl ketone.

M.p. 121-123 ° C (ethanol)

| Elem. Anal.($C_{20}H_{23}NO_2$ S) | | | |
|---|---|---|---|
| Calcd (%) | C : 70.35 | H : 6.79 | N : 4.10 |
| Found (%) | C : 70.31 | H : 6.78 | N : 4.06 |

Example 58. 3-(1,1-Dimethylethyl)-4-hydroxy-5-[N-(2-hydroxyethyl-N-ethylaminomethyl]phenyl 2-thienyl ketone hydrochloride.

M.p. 155-156 ° C (ethanol)

| Elem. Anal.($C_{20}H_{27}NO_3$ S • HCl) | | | |
|---|---|---|---|
| Calcd (%) | C : 60.36 | H : 7.09 | N : 3.52 |
| Found (%) | C : 60.18 | H : 7.21 | N : 3.47 |

Example 59. 3-(1,1-Dimethylethyl)-4-hydroxy-5-(N,N-di-n-butylaminomethyl)phenyl 2-thienyl ketone hydrochloride.

M.p. 107-108 ° C (ethanol/ether)

| Elem. Anal.($C_{24}H_{35}NO_2$ S • HCl) | | | |
|---|---|---|---|
| Calcd (%) | C : 65.80 | H : 8.28 | N : 3.20 |
| Found (%) | C : 65.63 | H : 8.48 | N : 3.09 |

Example 60. 3-(1,1-Dimethylethyl)-4-hydroxy-5-(N,N-di-isopropylaminomethyl)phenyl 2-thienyl ketone hydrochloride.

M.p. 155-156 ° C (ethanol/ether)

| Elem. Anal.($C_{22}H_{31}NO_2$ S • HCl) | | | |
|---|---|---|---|
| Calcd (%) | C : 64.45 | H : 7.87 | N : 3.42 |
| Found (%) | C : 64.32 | H : 7.90 | N : 3.27 |

Example 61. 3-(1,1-Dimethylethyl)-4-hydroxy-5-[N-(2-hydroxyethyl)-N-methylaminomethyl]phenyl 2-thienyl ketone benzenesulfonate.

M.p. 162-163 ° C (methanol/isopropyl ether)

| Elem. Anal.($C_{19}H_{25}NO_3S \bullet C_6H_6O_3S$) | | | |
|---|---|---|---|
| Calcd (%)<br>Found (%) | C : 59.38<br>C : 59.32 | H : 6.18<br>H : 6.21 | N : 2.77<br>N : 3.04 |

Example 62. 3-(1,1-Dimethylethyl)-4-hydroxy-5-[N-(2-acetyloxyethyl)-N-methylaminomethyl]phenyl 2-thienyl ketone.

M.p. 124-126° C (ethanol/water)

| Elem. Anal.($C_{21}H_{27}NO_4S$) | | | |
|---|---|---|---|
| Calcd (%)<br>Found (%) | C : 64.76<br>C : 64.72 | H : 6.99<br>H : 7.04 | N : 3.60<br>N : 3.67 |

Example 63. 3-(1,1-Dimethylethyl)-4-hydroxy-5-[N-(2-hydroxyethyl-N-methylaminomethyl]-4′-methylbenzophenone.

M.p. 131-132° C (ethanol/water)

| Elem. Anal.($C_{22}H_{29}NO_3$) | | | |
|---|---|---|---|
| Calcd (%)<br>Found (%) | C : 74.33<br>C : 74.25 | H : 8.22<br>H : 8.36 | N : 3.94<br>N : 4.17 |

Example 64. 3-(1,1-Dimethylethyl)-4-hydroxy-5-[N-(2-hydroxyethyl-N-methylaminomethyl)-4′-chlorobenzophenone.

M.p. 126-127° C (ethanol/water)

| Elem. Anal.($C_{21}H_{26}ClNO_3$) | | | |
|---|---|---|---|
| Calcd (%)<br>Found (%) | C : 67.10<br>C : 67.03 | H : 6.97<br>H : 6.97 | N : 3.73<br>N : 3.84 |

Example 65. 3-(1,1-Dimethylethyl)-4-hydroxy-5-(N-n-butyl-N-ethylaminomethyl)phenyl 2-thienyl ketone hydrochloride.

M.p. 140-142° C (acetone/n-hexane)

| Elem. Anal.($C_{22}H_{31}NO_2S \bullet HCl$) | | | |
|---|---|---|---|
| Calcd (%)<br>Found (%) | C : 64.45<br>C : 64.29 | H : 7.87<br>H : 8.00 | N : 3.42<br>N : 3.40 |

Example 66. 3-(1,1-Dimethylethyl)-4-hydroxy-5-(N,N-di-cyclohexylaminomethyl)phenyl 2-thienyl ketone.

M.p. 161-162 °C (ethanol)

| Elem. Anal.($C_{28}H_{33}NO_2S$) | | | |
|---|---|---|---|
| Calcd (%) | C : 74.13 | H : 8.66 | N : 3.09 |
| Found (%) | C : 74.00 | H : 8.76 | N : 2.96 |

Example 67. 3-(1,1-Dimethylethyl)-4-hydroxy-5-[N-n-butyl-N-(2-hydroxyethyl)aminomethyl]phenyl 2-thienyl ketone hydrochloride.

M.p. 125-128 °C (ethanol/ether)

| Elem. Anal.($C_{22}H_{31}NO_3S \bullet HCl$) | | | |
|---|---|---|---|
| Calcd (%) | C : 62.03 | H : 7.57 | N : 3.29 |
| Found (%) | C : 61.83 | H : 7.57 | N : 3.47 |

Example 68. 3-(1,1-Dimethylethyl)-4-hydroxy-5-(N,N-di-n-hexylaminomethyl)phenyl 2-thienyl ketone hydrochloride.

M.p. 106-108 °C (ethanol/ether)

| Elem. Anal.($C_{28}H_{43}NO_2S \bullet HCl$) | | | |
|---|---|---|---|
| Calcd (%) | C : 68.05 | H : 8.97 | N : 2.83 |
| Found (%) | C : 67.70 | H : 8.99 | N : 2.94 |

Example 69. 3-(1,1-Dimethylethyl)-4-hydroxy-5-(N-allyl-N-cyclohexylaminomethyl)phenyl 2-thienyl ketone hydrochloride.

M.p. 154-156 °C (ethanol/ether).

| Elem. Anal.($C_{25}H_{33}NO_2S \bullet HCl$) | | | |
|---|---|---|---|
| Calcd (%) | C : 67.02 | H : 7.65 | N : 3.13 |
| Found (%) | C : 66.95 | H : 7.62 | N : 3.07 |

Example 70. 3-(1,1-Dimethylethyl)-4-hydroxy-5-(N,N-di-n-octylaminomethyl)phenyl 2-thienyl ketone hydrochloride.

M.p. 90-91 °C (ether/n-hexane)

| Elem. Anal.($C_{32}H_{51}NO_2S \bullet HCl$) | | | |
|---|---|---|---|
| Calcd (%) | C : 69.85 | H : 9.52 | N : 2.55 |
| Found (%) | C : 69.75 | H : 9.39 | N : 2.51 |

Example 71. 3-(1,1-Dimethylethyl)-4-hydroxy-5-[N-cyclohexyl-N-(2-hydroxyethyl)aminomethyl]phenyl 2-thienyl ketone hydrochloride.

M.p. 156-157° C (isopropanol).

| Elem. Anal.($C_{24}H_{33}NO_3S \bullet HCl$) | | | |
|---|---|---|---|
| Calcd (%) | C : 63.77 | H : 7.58 | N : 3.10 |
| Found (%) | C : 63.50 | H : 7.55 | N : 3.03 |

Example 72. 3-(1,1-Dimethylethyl)-4-hydroxy-5-[N-(2-hydroxyethyl)-N-methylaminomethyl]-2′-fluorobenzophenone.

M.p. 87-89° C (ethanol/water)

| Elem. Anal.($C_{21}H_{26}FNO_3$) | | | |
|---|---|---|---|
| Calcd (%) | C : 70.17 | H : 7.29 | N : 3.90 |
| Found (%) | C : 69.99 | H : 7.27 | N : 4.04 |

Example 73. 3-(1,1-Dimethylethyl)-4-hydroxy-5-[N-(2-hydroxyethyl)-N-methylaminomethyl]benzophenone.

M.p. 118-119° C (ethanol/water)

| Elem. Anal.($C_{21}H_{27}NO_3$) | | | |
|---|---|---|---|
| Calcd (%) | C : 73.87 | H : 7.97 | N : 4.10 |
| Found (%) | C : 73.80 | H : 8.06 | N : 3.98 |

Example 74. 3-(1-Methylpropyl)-4-hydroxy-5-[N-(2-hydroxyethyl)-N-methylaminomethyl]benzophenone hydrochloride.

M.p. 67-70° C (ethanol/isopropyl ether)

| Elem. Anal.($C_{21}H_{27}NO_3 \bullet HCl \bullet H_2O$) | | | |
|---|---|---|---|
| Calcd (%) | C : 63.71 | H : 7.64 | N : 3.54 |
| Found (%) | C : 63.31 | H : 7.61 | N : 3.51 |

Example 75. 3-(1,1-Dimethylethyl)-4-hydroxy-5-[N-(2-hydroxyethyl)-N-methylaminomethyl]phenyl 2-thienyl ketone phosphate.

M.p. 137-140° C (ethyl acetate)

| Elem. Anal.($C_{19}H_{25}NO_3S \bullet H_3O_4P$) | | | |
|---|---|---|---|
| Calcd (%) | C : 51.23 | H : 6.34 | N : 3.14 |
| Found (%) | C : 51.09 | H : 6.44 | N : 2.68 |

Example 76. 3-Cyclohexyl-4-hydroxy-5-[N-(2-hydroxyethyl)-N-methylaminomethyl]benzophenone.

M.p. 92-93° C (isopropyl ether/n-hexane)

| Elem. Anal.($C_{23}H_{29}NO_3$) | | | |
|---|---|---|---|
| Calcd (%) | C : 75.17 | H : 7.95 | N : 3.81 |
| Found (%) | C : 75.18 | H : 8.24 | N : 3.67 |

Example 77. 3-(1-Methylethyl)-4-hydroxy-5-[N-methyl-N-(2-hydroxyethyl)aminomethyl]phenyl 2-thienyl ketone hydrochloride.

M.p. 125-128° C (isopropanol/ether)

| Elem. Anal.($C_{18}H_{23}NO_3S \bullet HCl \bullet \frac{1}{4} H_2O$) | | | |
|---|---|---|---|
| Calcd (%) | C : 57.74 | H : 6.60 | N : 3.74 |
| Found (%) | C : 57.76 | H : 6.71 | N : 3.81 |

Example 78. 3-(1-Methylpropyl)-4-hydroxy-5-[N-methyl-N-(2-hydroxyethyl)aminomethyl]phenyl 2-thienyl ketone hydrochlroide.

M.p. 114-117° C (isopropanol/ethyl acetate)

| Elem. Anal.($C_{19}H_{25}NO_3S \bullet HCl$) | | | |
|---|---|---|---|
| Calcd (%) | C : 59.44 | H : 6.83 | N : 3.65 |
| Found (%) | C : 59.30 | H : 7.13 | N : 3.73 |

Example 79. 3-(1,1-Dimethylethyl)-4-hydroxy-5-[N-n-butyl-N-(carbamoylmethyl)aminomethyl]phenyl 2-thienyl ketone .

M.p. 146-147° C (ether)

| Elem. Anal.($C_{22}H_{30}N_2O_3S \bullet 1/3\ H_2O$ ) | | | |
|---|---|---|---|
| Calcd (%) | C : 64.68 | H : 7.68 | N : 6.86 |
| Found (%) | C : 64.59 | H : 7.52 | N : 6.75 |

Example 80. 3-(1,1-Dimethylethyl)-4-hydroxy-5-[N-(2-hydroxyethyl)-N-methylaminomethyl]-2′-trifluoromethylbenzophenone hydrochloride.

M.p. 175-176° C (ethyl acetate/ether)

| Elem. Anal.($C_{22}H_{26}F_3NO_3S \bullet HCl$) | | | |
|---|---|---|---|
| Calcd (%)<br>Found (%) | C : 59.26<br>C : 59.37 | H : 6.10<br>H : 6.29 | N : 3.14<br>N : 3.13 |

Example 81. 3-(1,1-Dimethylethyl)-4-hydroxy-5-[N-(2-hydroxyethyl)-N-methylaminomethyl]-2′,6′-dichlorobenzophenone hydrochloride.

M.p. 122-124° C (ethyl acetate)

| Elem. Anal.($C_{21}H_{25}Cl_2NO_3S \bullet HCl$) | | | |
|---|---|---|---|
| Calcd (%)<br>Found (%) | C : 56.46<br>C : 56.16 | H : 5.87<br>H : 6.06 | N : 3.13<br>N : 3.10 |

Example 82. 3-(1,1-Dimethylethyl)-4-hydroxy-5-[N-(2-hydroxyethyl)aminomethyl]phenyl 2-thienyl ketone.

M.p. 145-146° C (ethyl acetate)

| Elem. Anal.($C_{18}H_{23}NO_3S$) | | | |
|---|---|---|---|
| Calcd (%)<br>Found (%) | C : 64.84<br>C : 64.81 | H : 6.95<br>H : 7.14 | N : 4.20<br>N : 3.81 |

Example 83. 5-(N-Carboxymethyl-N-methylaminomethyl)-3-(1,1-dimethylethyl)-4-hydroxyphenyl 2-thienyl ketone.

M.p. 180-182° C (ethanol/water)

| Elem. Anal.($C_{19}H_{23}NO_4S$) | | | |
|---|---|---|---|
| Calcd (%)<br>Found (%) | C : 63.14<br>C : 62.86 | H : 6.41<br>H : 6.57 | N : 3.88<br>N : 3.73 |

Example 84. 3-(1,1-Dimethylethyl)-4-hydroxy-5-[N-(3-hydroxypropyl)-N-methylaminomethyl]phenyl 2-thienyl ketone hydrochloride.

M.p. 146-148° C (ethanol/isopropyl ether)

| Elem. Anal.($C_{20}H_{27}NO_3S \bullet HCl$) | | | |
|---|---|---|---|
| Calcd (%)<br>Found (%) | C : 60.36<br>C : 60.19 | H : 7.09<br>H : 7.17 | N : 3.52<br>N : 3.68 |

Test Example.

With respect to representative examples of the compounds of the present invention, their usefulness was tested. The result of the pharmacological experiment is given in Table 1. The drug tested was dissolved or suspended in 0.5% methylcellulose (MC) and subjected to the test.

(1) Inhibitory Action to Carrageenin Edema.

Seven male rates (SD strain; 6 weeks age) were used in a group. The drug was administered per os and, after one hour, 0.1 ml of 0.5% λ-carrageenin solution was injected subcutaneously into right hind paw. After three hours, the volume of the right hind paw was measured with a plethysmometer (UGO-BASILE). From the value was deducted that before the carrageenin injection and the degree of swelling was calculated. Out of the degree of swelling of the drug-treated group to that of the control group, inhibitory rate was obtained and $ED_{30}$ was calcualted.

(2) Inhibitory Action to Acetate Acid Writhing.

Six male mice (ddY strain; five weeks age) were used in a group. The drug was administered per os and, after one hour, 0.6% acetate acid was intraperitoneally injected at the dose of 0.1 ml/10 g and the total writhings within 20 minutes thereafter were counted. Out of the writhing numbers of the drug-treated group to that of the control group the inhibitory rate was obtained and $ED_{50}$ was calculated.

(3) Inhibitory Action to Pain by Heat Stimulation.

Ten male mice (ddY strain; five weeks age) were used in a group. Radiated heat (250 watts; 4 cm distance from the heat source) was applied to the end of the tail of mice and the animals which showed a tail flick within 4 to 7 seconds were selected and subejcted to the experiment. Drug was administered per os and the time of appearance of the tail flick after 5, 15, 30, 45 and 60 minutes was measured. The mice which needed longer time than the sum of $3\sigma$ and the average time (needed for showing tail flick before administration of the drug) were judged to be analgesically positive. The maximum positive rate among the analgesically positive rates in each measuring time was defined as the analgesically positive rate at that dose and $ED_{50}$ was calculated therefrom.

(4) Stomach Disorder.

Ten male rats (SD strain; five weeks age) were used in a group. The drug dissolved or suspended in physiolo gical saline solution was administered to them per os. Then the animals were fasted for 17 hours, then 0.5 ml of 5% pontamine sky blue solution was injected intravenously and, after 10 minutes, they were sacrificed with ether. Stomach was taken out from the animals, about 8 ml of 5% formaline solution was injected into the stomach to subject to a semi-fixing and the presence of the edema was checked using a stereoscopic microscope. Rats in which even one ulcer was observed was judged to be positive in ulcer generation and $UD_{50}$ was calculated by a provit method.

(5) Acute Toxicity

Four male rats (SD strain; six weeks age) were used in a group. The drug was given per os and, after 7 days, their death or alive was checked and $LD_{50}$ was calculated by a provit method.

Table 1

| Tests | Compd of Example 4 | R-830 | Indomethacin | Thiaramide |
|---|---|---|---|---|
| (1) $ED_{30}$ (mg/kg) | 9.8 | 5.6 | 0.8 | 112.2 |
| (2) $ED_{50}$ (mg/kg) | 16.4 | 22.4 | 2.1 | 27.3 |
| (3) $ED_{50}$ (mg/kg) | 47.0 | *(1) | *(2) | 108.5 |
| (5) $UD_{50}$ (mg/kg) | 146.4 | 11.8 | 2.4 | 277.6 |
| (4) $LD_{50}$ (mg/kg) | >1000 | 1625* | 29* | 4700* |
| Safety Region ($UD_{50}/ED_{50}$) | 14.9 | 2.1 | 3.0 | 2.5 |

*: values given in literatures
*(1): no action at 500 mg/kg
*(2): no action at 20 mg/kg

It is apparent from the above table that the compounds of the present invention exhibits two characteristic features of basic and acidic antiinflammatory agents and showed strong analgesic and antiinflammatory activities.

Thus they exhibited analgesic activity (which is as strong as codeine) not only to inflammatory pain (such as acetic acid writhing) but also to noninflammaotry one by heat stimulation and, at the same time, they exhibited inhibitory action to carrageenin edema.

The compounds of the present invention show excellent analgesic and antiinflammatory actions which are not achieved in the conventional drugs and, moreover, their ulcer generation action and toxicity are very low. Accordingly, their safety region is quite broad and, therefore, they can be used as a safe remedies for various pains, arthiritis, rheumatism, and upper airway inflammation of mammalia including human being.

## Claims

(1) Acylphenol derivatives represented by the following general formula (I) and physiologically-acceptable salts thereof.

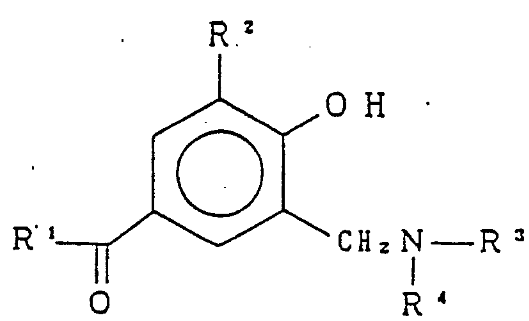

( I )

wherein $R^1$ is cycloalkyl, substituted or unsubstituted aryl or

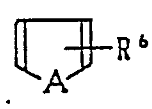

in which A is oxygen, sulfur or $NR^5$ ($R^5$ is hydrogen or alkyl) and $R^6$ is hydrogen, alkyl or halogen; $R^2$ is alkyl or cycloalkyl; and $R^3$ and $R^4$, which are same or different, are hydrogen, substituted or unsubstituted alkyl, alkenyl, alkynyl or cycloalkyl or $R^3$ and $R^4$ are bonded forming cyclic amino together with the adjacent nitrogen atom.